# EUROPEAN PATENT APPLICATION

(11) **EP 1 795 202 A1**
(43) Date of publication of application: **13.06.2007**
(21) Application number: 05077820.8
(22) Date of filing: 07.12.2005
(51) Int. Cl.: A61K 33/08, A61P 3/02

(54) **Preparation for treatment of mineral deficiency**

(71) Applicant: Nederlandse Organisatie voor Toegepast-Natuurwetenschappelijk Onderzoek TNO, 2628 VK Delft (NL)
(72) Inventor: Slaghek, Theodoor Maximiliaan, 3042 HT Rotterdam (NL); Batenburg, Lawrence Fabian, 5611 CK Eindhoven (NL); Fischer, Hartmut Rudolf, 5731 TP Mierlo (NL)
(74) Representative: Winckels, Johannes Hubertus F.

(57) **Abstract**

The invention relates to a preparation having an improved taste, as well as to the use of said preparation for treatment of mineral deficiency. In accordance with the invention, it has been found possible to effectively mask the bad taste of even such notorious minerals as iron. As a result, patient compliance may be increased significantly by prescription of a preparation according to the invention, thereby increasing effective uptake of the deficient mineral.

## Description

The invention relates to a preparation as well as to the use of said preparation for treatment of mineral deficiency.

The term mineral deficiency means a condition where the concentration of any one of the minerals essential to human and/or animal health is abnormally low in the body. In some cases, an abnormally low mineral concentration is defined as that which leads to an impairment in a function dependent on the mineral. In other cases, the convention may be to define an abnormally low mineral concentration as a level lower than that found in a specific healthy population.

The mineral content of the body may be measured by testing samples of blood plasma, red blood cells, or urine. In the case of calcium and phosphate deficiency, the diagnosis may also involve taking X-rays of the skeleton. In the case of iodine deficiency, the diagnosis may include examining the patient's neck with the eyes and hands. In the case of iron deficiency, the diagnosis may include the performance of a stair-stepping test by the patient. Since all the minerals serve strikingly different functions in the body, the tests for the corresponding deficiency are markedly different from each other.

The class of mineral nutrients typically includes all inorganic elements or inorganic molecules that are required for life. As far as human nutrition is concerned, the inorganic nutrients include water, sodium, potassium, chloride, calcium, phosphate, sulfate, magnesium, iron, copper, zinc, manganese, iodine, selenium, and molybdenum. Some of the inorganic nutrients, such as water, do not occur as single atoms, but occur as molecules. Other inorganic nutrients that are molecules include phosphate, sulfate, and selenite. There is some evidence that other inorganic nutrients, such as chromium and boron, play a part in human and animal health, but their role is not well established. Fluoride has been proven to increase the strength of bones and teeth, but there is little or no reason to believe that it is needed for human and animal life.

Iron is an essential component of hemoglobin, the oxygen carrying complex (pigment) in the blood. Iron is normally obtained through the food in the diet. Iron-deficient people tire easily because their bodies are starved for oxygen. Iron is also part of myoglobin. Myoglobin helps muscle cells store oxygen. Without enough iron, the body's fuel cannot be properly synthesized. Iron Deficiency Anemia (also called IDA) is a condition where a person has inadequate amounts of iron to meet body demands. It is a decrease in the amount of red cells in the blood caused by having too little iron. IDA is usually caused by a diet insufficient in iron or from blood loss. Blood loss can be acute as in hemorrhage or trauma or long term as in heavy menstruation. Iron deficiency anemia is the most common form of anemia. About 20% of women, 50% of pregnant women, and 3% of men are iron deficient.

Treatment of mineral deficiency typically, in less severe cases, entails prescription of special diets based on intake of foods which have a high content of the deficient mineral. In more severe cases, treatment usually includes administration of food supplements. These may take the form of so-called multivitamin or multimineral supplements, containing many vitamins and/or minerals, typically in the Recommended Dietary Allowance (which may also be referred to as Recommended Daily Allowance, RDA). However, mineral supplements containing only one, or a few, specific minerals are also commonly prescribed. For instance, iron supplements can be taken over several months to increase iron levels in the blood. Iron supplements can cause irritation of the stomach and discoloration of bowel movements.

A disadvantage of many mineral supplements is that they produce an unpleasant taste, either during or immediately after oral administration. For some minerals this may be a bitter taste, for others a metallic taste. Many mineral supplements have the form of tablets or capsule since it provides for easy and cost-effective administration. Upon oral administration of such dosage forms, the unpleasant taste may be detected when the bad tasting ingredient comes into contact with the taste buds, particularly by overlong holding the dosage form in the mouth, by inadvertent chewing, or by some other release of the bad tasting ingredient.

Patients give many different reasons for their poor compliance with administration regimen of drugs, including mineral supplements. Examples are unattractive appearance, overlarge size, bad taste or fear that an unchewed dosage form may get stuck in the throat. Patients who have difficulties with oral dosage forms often exhibit a gag reflex which prevents adequate oral administration. This phenomenon occurs more often, but not exclusively, in children.

Although chewable dosage forms have been developed which may overcome some of these problems, many pharmaceutical agents have such a bad taste, e.g. bitter or metallic, that it cannot be tolerated when chewed. The prolonged exposure of the patient to the unpleasant taste may also cause compliance problems. Conventionally, attempts have been made to address this issue by incorporating sweeteners or flavoring agents as taste masking agents in oral dosage forms. These agents provide a secondary flavor which is intended to overwhelm any unpleasant taste of the pharmaceutical agent. Unfortunately, for many powerfully bad tasting pharmaceuticals this approach is inadequate.

In accordance with the present invention, it has surprisingly been found that the unpleasant taste of various cationic minerals can be masked in a highly effective manner by incorporating the mineral into a layered double hydroxide. The invention thus provides a preparation comprising a layered double hydroxide into which one or more minerals are incorporated, as well as a method for treating a condition of mineral deficiency in a human or animal in need thereof comprising administering an effective amount of a layered double hydroxide into which one or more minerals incorporated.

By incorporating a mineral into a layered double hydroxide, it has been found possible to effectively mask the bad taste of even such notorious minerals as iron. As a result, patient compliance may be increased significantly by prescription of a preparation according to the invention, thereby increasing effective uptake of the deficient mineral. Other advantages of the invention will become clear from the following detailed description of the various embodiments of the invention.

Layered double hydroxides are clays of an anionic nature which may be found in nature or prepared synthetically. They consist of small crystalline sheets of dimensions of a few nanometers up to a few tens of nanometers, between which anions are located. By these anions are meant anions other than hydroxyl groups. For a description of possible methods of preparation for a synthetic layered double hydroxide, reference is made to U.S. Patents 3,539,306 and 3,650,704.

A layered double hydroxide according to the invention into which one or more minerals are incorporated preferably has the formula

[(M^{II})₍₁₋ₓ₎ (M^{III})ₓ (OH)₂] [A_{x/y}^{y-}.n H₂O] (I),

or

[(M^{I}M^{III}₂] [A^{y-}._{1/n}]·nH₂O (II),

wherein M^{I}, M^{II} and M^{III} are respectively one or more mono-, bi- and tri-valent metal cations.

In formula (I), M^{II} can be one or more bivalent metals, and M^{III} can be one or more trivalent metals, where x is a number between 0.15 and 0.5, y is 1 or 2, n is a number from 1 to 10, and A is an anion selected from the group consisting of Cl⁻, Br-, NO₃⁻, PO₄³⁻, SO₄²⁻, CO₃²⁻, and combinations thereof.

The bivalent cation is preferably selected from the group of bivalent magnesium, chromium, zinc, germanium, nickel, iron, copper, cobalt, calcium, molybdenum, vanadium and manganese ions and combinations of these bivalent cations. The trivalent cation is preferably selected from the group of trivalent aluminum, chromium, iron, cobalt, molybdenum, vanadium and manganese ions and combinations of these trivalent cations.

In a preferred embodiment, the bivalent cation (M^{II})₍₁₋ₓ₎ is a combination of magnesium and a bivalent mineral in which an intended patient is deficient. The stoichiometric ratio of magnesium to the bivalent deficient mineral is preferably between 1:0 and 0:1, more preferably between 0.5:0.5 and 0.1:0.9. Likewise, the trivalent cation (M^{III})ₓ is preferably a combination of aluminum and a trivalent mineral in which an intended patient is deficient. The stoichiometric ratio of aluminum to the trivalent deficient mineral is preferably between 1:0 and 0:1, more preferably between 0.5:0.5 and 0.1:0.9.

In formula (II), M^{I} can be one or more mono-valent metals, and M^{III} can be one or more trivalent metals, where n is a number from 1 to 10, and A is an anion selected from the group consisting of Cl⁻, Br⁻, NO₃⁻, PO₄³⁻, SO₄²⁻, CO₃²⁻, and combinations thereof.

The mono-valent cation is preferably selected from the group of mono-valent lithium, sodium, potassium, and silver and combinations of these mono-valent cations. The trivalent cation is preferably selected from the group of trivalent aluminum, chromium, iron, cobalt, molybdenum, vanadium and manganese ions and combinations of these trivalent cations.

In accordance with the invention it is possible to treat both animals and humans for a mineral deficiency. It is preferred, however, that humans are treated.

A layered double hydroxide having the formula (I), as described above, may be prepared analogous to any known convenient manner for preparing layered double hydroxides. An example of a suitable method is derived from the method disclosed in US patent 3,539,306, wherein hydrotalcite having the formula Mg₆Al₂(OH)₁₆CO_{3.}4H₂O is prepared by mixing an aluminum component with a magnesium component in an aqueous medium in the presence of carbonate ions at a suitable pH, preferably at least 8, and recovering the resultant precipitate, wherein the aluminum component and the magnesium component have suitable counter ions to obtain the desired anion A in the layered double hydroxide. In accordance with the invention, the aluminum component and/or the magnesium component may completely or partially be replaced by an appropriate component of the desired trivalent and/or bivalent cation, respectively, to yield a layered double hydroxide having formula (I).

A preparation according to the invention can be used for incorporation into medicinal compositions, such as pharmaceutical compositions or drugs, food supplements, or food or feed products. In these applications, a preparation according to the invention will typically be combined with other ingredients typically employed in such products.

When the preparation is used in a medicinal composition or a food supplement, this composition or supplement preferably comprises, in addition to the above described layered double hydroxide, a pharmaceutically acceptable excipient. Depending on the oral dosage form, the excipient may be chosen from the classes of fillers, lubricants, sweeteners, flavorants, colorants, antioxidants, coating materials, and other excipients known to the person skilled in the art, and combinations thereof.

When a preparation according to the invention is used in a food or feed product, it will preferably be mixed with the other ingredients of the end product in such a way to provide a substantially homogenous incorporation of the preparation according to the invention in the product.

In a preferred embodiment, in particularly wherein a bivalent cationic mineral, which may exist in bivalent or trivalent form (such as iron) is to be administered, a preparation according to the invention also comprises an antioxidant. Preferred examples of suitable antioxidants include, but are not limited to, ascorbic acid and chelating agents, as used in iron (both ferri and ferro) containing fruit or fruit like juices or iron based supplements applicable for anaemia like dextran, dextransulphate, carboxydextran, fumarate, and gluconate and the like. The presence of an antioxidant may help preventing undesired oxidation of bivalent cations to trivalent cations. This is particularly preferred for treating iron deficiencies, wherein iron(II) is much more efficacious than iron(III). In accordance with this embodiment, the antioxidant is preferably present in an amount of 0.001 to 0.5 wt.%, based on the weight of the end product e.g. fruit-like juice or iron based supplement.

In preferred embodiment the bivalent cation and the antioxidant are in a molar ratio that allows an effective prevention of oxidation of the bivalent cation. For example, in the case of iron(II) and ascorbic acid the weight ratio is preferably between 0.03: 1 and 5:1, more preferably between 0.1:1 and 3:1.

Preferably, when applied in a vehicle, the preparation according to the invention provides at least 5 %, more preferably 100 % and most preferably 1000 % of the recommended daily dosage of the incorporated mineral per 100 mL of vehicle. In case essential vitamins such as ascorbic acid or tocopherol are used as antioxidant, the preparation also provides at least 5 %, more preferably 100 % and most preferably 1000 % of the recommended daily dosage of the respective vitamin per 100 mL of vehicle.

The invention will now be elucidated by the following, non-restrictive examples.

### Example 1. Synthesis of Mg-Fe(III)-Cl LDH in a Fe:Mg ratio of 4:1

75.12 g of NaOH was weighed under nitrogen atmosphere in a 5 L three-necked flask. Then, 937 mL of boiled demineralized water (cooled to about 40 °C) was added under nitrogen atmosphere. 141.75 g of MgCl₃·6 H₂O was weighed in a beaker and dissolved in 875 g of boiled demineralized water (cooled) after which 47.05 g of FeCl₃·6 H₂O was added and the mixture was stirred under nitrogen. The brown solution was added to a dropping funnel and fixed to the 5 L three-necked flask in a nitrogen atmosphere. While stirring intensively the Mg-Fe solution was added dropwise until the reaction mixture had a pH of 9.5. Then, the solution was heated for 6 hours at 100 °C. The solution was filtered and washed with demineralized water until all NaCl had been removed. The product was freeze-dried.

### Example 2. Synthesis of Mg-Fe(III)-Cl LDH in a Fe:Mg ratio of 2:1

11.56 g of NaOH was weighed under nitrogen atmosphere in a 500 mL three-necked flask. Then, 145.65 mL of boiled demineralized water (cooled to about 40 °C) was added under nitrogen atmosphere. 19.72 g of MgCl₃ 6 H₂O was weighed in a beaker and dissolved in 146 g of boiled demineralized water (cooled) after which 13.10 g of FeCl₃ ·6 H₂O was added and the mixture was stirred under nitrogen. The brown solution was added to a dropping funnel and fixed to the 500 mL three-necked flask in a nitrogen atmosphere. While stirring intensively the Mg-Fe solution was added dropwise until the reaction mixture had a pH of 9.5. Then, the solution was heated for 6 hours at 100 °C. The solution was filtered and washed with demineralized water until all NaCl has been removed. The product was freeze-dried.

### Example 3. In-situ synthesis of Mg-Fe(III)-Cl LDH in a Fe:Mg ratio of 4:1 with vitamin C

41.6 g of NaOH was weighed under nitrogen atmosphere in a 2 L glass or steel reactor. Then, 467 g of boiled demineralized water (cooled to about 40 °C) was added under nitrogen atmosphere. Then, 17.6 g of vitamin C was added. 70.2 g of MgCl₃ · 6 H₂O was weighed in a beaker and dissolved in 430 g of boiled demineralized water (cooled) after which 23.4 g of FeCl₃ · 6 H₂O was added and the mixture was stirred under nitrogen. The brown solution was added to a dropping funnel and fixed to the 2 L reactor in a nitrogen atmosphere. While stirring intensively the Mg-Fe solution was added dropwise until the reaction mixture had a pH of 9.5. Then, the solution was heated for 6 hours at 100 °C. The solution was filtered and washed with demineralized water until all NaCl had been removed. The product was freeze-dried.

### Analysis by X-ray diffraction, thermal gravimetrical analysis, and infrared spectroscopy showed the presence of vitamin C in the Mg-Fe(III)-Cl LDH

### Example 4. In vitro application of pharmaceutical composition

In duplicate experiments, 1 g of a product prepared according to Example 1 (containing 220 g Fe(III) per kg of hydrotalcite) and 4 g of vitamin C were mixed with artificial saliva and drinking water. The total intake in the gastric compartment of the model was 200 g.

### Experiments were performed in TIM (TNO's Intestinal Model). For a discussion of this model, see inter alia the following publications:

Freidig, A. and Verwei, M. (2004). Integration of in vitro data in kinetic models for pharmaceuticals and nutrients. Netherlands Centre Altern. Animal Use Newsletter 16: 1-3.
Havenaar, R. and Minekus, M. (1996). Simulated assimilation. Dairy Industries International 61 (9): 17-23.
Minekus, M. and Havenaar, R. (1998). Reactor system. European Patent No. 0642382. European Patent Bulletin 98/07, Art. 97(4) and (5) EPC, dated 11.02.98.
Minekus, M. and Havenaar, R. (1996). In vitro model of an in vivo digestive tract. United States Patent; nr. 5,525,305, dated June 11, 1996.
Minekus, M., Marteau, P., Havenaar, R. and Huis in 't Veld, J.H.J. (1995). A multi compartmental dynamic computer-controlled model simulating the stomach and small intestine. Alternatives to Laboratory Animals (ATLA) 23: 197-209.
Smeets-Peeters, M.J.E., Watson, T., Minekus, M., Havenaar, R. (1998). A review of the physiology of the canine digestive tract related to the development of in vitro systems. Nutrition Research Reviews 11: 45-69.

These experiments were performed under the average physiological conditions of the gastrointestinal tract as described for humans. These conditions include especially the dynamics of gastric emptying and intestinal transit times, the gastric and the intestinal pH values, and the composition and activity of the secretion products. The digested and dissolved low-molecular compounds are dialysed continuously from the jejunum and ileum compartments of the model via hollow fibre membrane systems. The experiments were performed during 300 minutes, resulting in a dialysate of 5 L. In the dialysate on average 19 mg/L of soluble iron was measured (AAS) (atomic absorption spectroscopy).

### Example 5. In vitro application of pharmaceutical composition

A syrup containing 60 gram carbohydrate, 0.3 grams protein, 0 grams fat and 0.5 gram dietary fiber, 100 mg Vitamin C (100 % of the daily intake) and 0.2 gram iron containing hydrotalcite prepared according to Example 1 (220 iron mg/g) was made. 100 ml of the syrup was introduced in the gastric compartment of the TIM Model. The experiments in TIM (TNO's Intestinal Model) were performed under the average physiological conditions of the gastrointestinal tract as described for humans. These conditions include especially the dynamics of gastric emptying and intestinal transit times, the gastric and the intestinal pH values, and the composition and activity of the secretion products. The digested and dissolved low-molecular compounds are dialyzed continuously from the jejunum and ileum compartments of the model via hollow fiber membrane systems. The experiments were performed during 300 minutes, resulting in a dialysate of 5 L. In the dialysate on average 3.1 mg/L of soluble iron was measured (AAS) (atomic absorption spectroscopy). In total 15.5 (51 * 3.1 mg soluble iron per 1) was bioavailable resembling approximately 100 % of the daily iron intake.

## Claims

1. Preparation comprising a layered double hydroxide having the formula
[(M^{II})₍₁₋ₓ₎(M^{III})ₓ(OH)₂] [A_{x/y}^{y-}.n H₂O] (I),
or
[(M^{I}M^{III}₂][A^{y-}._{1/n}]· ₙH₂O (II),
wherein in formula (I), M^{II} represents one or more bivalent metals, and M^{III} represents one or more trivalent metals, and x is a number between 0.15 and 0.5, y is 1 or 2, n is a number from 1 to 10, and A is an anion selected from the group consisting of Cl⁻, Br⁻, NO₃⁻, PO₄³⁻, SO₄²⁻, CO₃²⁻, and combinations thereof; and
wherein in formula (II), M^{I} represents one or more mono-valent metals, and M^{III} represents one or more trivalent metals, and n is a number from 1 to 10, and A is an anion selected from the group consisting of Cl⁻, Br⁻, NO₃⁻, PO₄³⁻, SO₄²⁻, CO₃²⁻, and combinations thereof.

2. Preparation according to claim 1 for treatment of mineral deficiency.

3. Preparation according to claim 2 for treatment of a deficiency of a cationic mineral.

4. Preparation according to claim 3, wherein the cationic mineral is chosen from the group consisting of calcium, chromium, germanium, iron, cobalt, copper, manganese, molybdenum, vanadium and zinc cations.

5. Preparation according to any of the preceding claims, wherein the bivalent cation is preferably selected from the group of bivalent magnesium, chromium, zinc, germanium, nickel, iron, copper, cobalt, calcium, molybdenum, vanadium and manganese ions and combinations of these bivalent cations.

6. Preparation according to any of the preceding claims, wherein the trivalent cation is preferably selected from the group of trivalent aluminum, chromium, iron, cobalt, molybdenum, vanadium and manganese ions and combinations of these trivalent cations.

7. Preparation according to any of the preceding claims, wherein M^{II} represents a combination of magnesium and a bivalent mineral in which an intended patient is deficient.

8. Preparation according to claim 7, wherein the stoichiometric ratio of magnesium to the bivalent deficient mineral is preferably between 1:0 and 0:1, more preferably between 0.5:0.5 and 0.1:0.9.

9. Preparation according to any of the claims 1-6, wherein M^{III} represents a combination of aluminum and a trivalent mineral in which an intended patient is deficient.

10. Preparation according to claim 9, wherein the stoichiometric ratio of aluminum to the trivalent deficient mineral is preferably between 1:0 and 0:1, more preferably between 0.5:0.5 and 0.1:0.9.

11. Preparation according to any of the claims 7-8, wherein the bivalent cation is Fe²⁺, and further comprising ascorbic acid.

12. Medicinal product, food supplement, or food or feed product comprising a preparation according to any of the preceding claims.

13. Medicinal product according to claim 12 further comprising a pharmaceutically acceptable excipient.

14. Medicinal product according to claim 13, wherein the excipient is chosen from the classes of fillers, lubricants, sweeteners, flavorants, colorants, antioxidants, coating materials, and combinations thereof.

15. Method for treating a condition of mineral deficiency in a human or animal in need thereof comprising administering an effective amount of a layered double hydroxide having the formula
[(M^{II})₍₁₋ₓ₎ (M^{III})ₓ (OH)₂] [A_{x/y}^{y-}.n H₂O] (I),
or
[(M^{I}M^{III} ₂] [A^{y-}._{1/n}]·nH₂O (II),
wherein in formula (I), M^{II} represents one or more bivalent metals, and M^{III} represents one or more trivalent metals, and x is a number between 0.15 and 0.5, y is 1 or 2, n is a number from 1 to 10, and A is an anion selected from the group consisting of Cl⁻, Br⁻, NO₃⁻, PO₄³⁻, SO₄²⁻, CO₃²⁻, and combinations thereof; and
wherein in formula (II), M^{I} represents one or more mono-valent metals, and M^{III} represents one or more trivalent metals, and n is a number from 1 to 10, and A is an anion selected from the group consisting of Cl⁻, Br⁻, NO₃⁻, PO₄³⁻, SO₄²⁻, CO₃²⁻, and combinations thereof.

16. Method according to claim 15, wherein the mineral deficiency is a cationic mineral deficiency.

17. Method according to claim 16, wherein the cationic mineral is chosen from the group consisting of calcium, chromium, germanium, iron, cobalt, copper, manganese, molybdenum, vanadium and zinc cations.

18. Method according to any of the claims 15-17, comprising administering an effective amount of a pharmaceutical preparation according to any of the claims 1-14.

19. Use of a preparation according to any of the claims 1-14 for the manufacture of a medicament for treatment of mineral deficiency.
